**Europäisches Patentamt**

**European Patent Office**

**Office européen des brevets**

(11) Veröffentlichungsnummer: **0 051 786**
**A1**

## (12) EUROPÄISCHE PATENTANMELDUNG

(21) Anmeldenummer: **81108892.1**

(22) Anmeldetag: **24.10.81**

(51) Int. Cl.³: **A 01 N 25/24**, A 61 K 7/40

(30) Priorität: **06.11.80 DE 3041814**

(43) Veröffentlichungstag der Anmeldung: **19.05.82**
**Patentblatt 82/20**

(84) Benannte Vertragsstaaten: **DE FR GB IT**

(71) Anmelder: **BAYER AG, Zentralbereich Patente, Marken und Lizenzen, D-5090 Leverkusen 1, Bayerwerk (DE)**

(72) Erfinder: **Von Bittera, Miklos, Max-Scheler-Strasse 7, D-5090 Leverkusen 3 (DE)**
Erfinder: **Stendel, Wilhelm, Dr., In den Birken 55, D-5600 Wuppertal 1 (DE)**

(54) **Formulierung zur Bekämpfung von tierischen Ektoparasiten, insbesondere Insekten und Acarinen.**

(57) Diese Formulierungen bestehen aus 0,1–20%, vorzugsweise 0,1–5% eines insektiziden Wirkstoffes;

1–40%, vorzugsweise 1–20% eines Gel-, Lack- oder Film-bildenden Polymers, das sowohl in Wasser, in organischen Lösungsmitteln oder in Mischungen aus organischen Lösungsmitteln und Wasser löslich oder anquellbar ist;

40–98%, vorzugsweise 60–90% Wasser oder einem organischen Wasser/Lösungsmittelgemisch oder einem organischen Lösungsmittel;

0,1–10% verschiedener Additiva wie Spreitmitteln, Antioxydantien, Farbstoffen, Weichmachern usw.

0051786

BAYER AKTIENGESELLSCHAFT    . 5090 Leverkusen, Bayerwerk

Zentralbereich
Patente, Marken und Lizenzen  Ad-by-c     5. 11. 80

Formulierung zur Bekämpfung von tierischen Ektoparasiten,
insbesondere Insekten und  Acarinen

Die Erfindung betrifft neue Formulierungen zur Bekämpfung tierischer Ektoparasiten, (Insekten und
Acarinen). Die Formulierungen zeichnen sich durch
eine stark ausgeprägte Residualwirkung über einen
längeren Zeitraum aus.

Es ist in der Vergangenheit bereits wiederholt versucht worden bei insektiziden Substanzen z.B. insektiziden Phosphorsäureestern durch spezielle Formulierungen den Zeitraum der Wirksamkeit zu verlängern,
d.h. eine gute Residualwirkung zu erzielen. Dieses
geschieht beispielsweise dadurch, daß flüchtige
insektizide Verbindungen mikroverkapselt werden. Auch
das Einarbeiten von Insektiziden in Polymere, aus
denen sie über einen längeren Zeitraum verdampfen, wird
in der Praxis durchgeführt, z.B. bei Hundehalsbändern
oder bei Fliegen-Strips , die beispielsweise O,O-Dimethyl-O-(2,2-dichlorvinyl)-phosphorsäureester als

Le A 20 522

- 2 -

**0051786**

Wirkstoff enthalten. Zur Bekämpfung von tierischen Ektoparasiten wie Insekten und Acarinen am Großtier z.B. Rind, Pferd, Schaf usw. sind solche Verfahren bisher nur selten und mit ungenügendem Erfolg zum Einsatz gekommen.

So wurden u.a. insektizide Ohrmarken gegen Zecken, die das Ohr bevorzugen, geprüft. (E.A.Ahrens et al., J. of Econom. Entomology, 70, 581-585).

Gegen Hydrotea irritans wurden bei Schafen insektizide Teerformulierungen eingesetzt, die gegenüber der Reinsubstanz eine verlängerte Wirksamkeit aufwiesen (R.N. Titchener, A.D. Herlyn and I.W. Newbold, Proceedings of the 8th Insecticide Conference 1975, 533-538).

Des weiteren sind Repellent-Formulierungen mit thermoplastischen Harzen beschrieben worden, die, auf die Haut aufgebracht, die Repellent-Wirkung verlängern (GS-PS 1.322.805).

Insektizide Formulierungen mit N-Vinyl-Lactam-Polymeren oder Vinylacetat-Crotonsäure-Copolymeren zur Anwendung bei Mensch und Tier werden ebenfalls in der Literatur beschrieben (siehe z.B. US-PS 3.301.808 oder GB-PS 10427.451).

Zur Anwendung am Tier wurde auch eine insektizide Formulierung mit Haftkleberzusatz beschrieben (DE-OS 26 58 725). Das in der genannten DE-OS beschriebene

Le A 20 522

Verfahren ist auf die Bekämpfung symboviner Fliegen gerichtet, weil speziell beim Rind dieses Indikationsgebiet in letzter Zeit große Bedeutung erlangt hat. Hierfür sind vor allem neue Methoden der Massenhaltung von Rindern (feed lots usw.) und die bei Einsatz des gegen Fliegen stark wirksamen Stoffes DDT bei schlachtbaren Tieren auftretenden Probleme verantwortlich.

Obwohl eine Reihe von Fliegenmitteln für Großtiere zur Zeit erhältlich ist, war die wirksame und vor allem dauerhafte Bekämpfung der symbovinen Fliegen beim Rind bis jetzt immer noch ein ungelöstes Problem. Die Ursache ist in dem ungenügenden Residualeffekt der bisher gegen symbovine Fliegen eingesetzten Präparate auf dem Rind zu suchen. Somit ist das Problem der Fliegen-Bekämpfung beim Rind weniger ein Problem des Wirkstoffeinsatzes als vielmehr einer geeigneten langwirksamen Formulierung. Ähnliche Probleme treten bei anderen Nutztieren auf.

Eine langwirkende Formulierung sollte sowohl auf nassen wie auf trockenen Tieren angewendet werden können. Dies ist insofern wichtig, als in tropischen Ländern die Rinder in der Regel zur Zeckenbekämpfung gebadet (=gedipt) oder gesprüht (=gesprayt) werden. Im Anschluß an dieses Bad ist es vorteilhaft, die Tiere auch noch mit einem Schutz gegen symbovine Fliegen zu versehen. Zum anderen sollte eine solche Formulierung auch auf trockenen Tieren anzuwenden sein.

Le A 20 522

Bei den zuvor zitierten insektiziden Formulierungen zum Auftragen auf das Haarkleid ist eine Anwendung aber nur bei trockenen Tieren möglich. Die Langzeitwirkung ist dabei nicht ausreichend.

Es wurden nun Formulierungen mit ektoparasitizider insbesondere insektizider und/oder gegebenenfalls Repellent-Wirkung gefunden, die eine ausgezeichnete Residualwirkung aufweisen, wenn sie auf das naße oder trockene Haarkleid von Großtieren aufgetragen werden.

Diese erfindungsgemäßen Formulierungen bestehen aus

0,1-20 %, vorzugsweise 0,1-5 % eines insektiziden Wirkstoffes;

1-40 %, vorzugsweise 1-20 % eines Gel-, Lack- oder Film-bildenden Polymers, das sowohl in Wasser, in organischen Lösungsmitteln oder in Mischungen aus organischen Lösungsmitteln und Wasser löslich oder anquellbar ist;

40-98 %, vorzugsweise 60-90 % Wasser oder einem organischen Wasser/Lösungsmittelgemisch oder einem organischen Lösungsmittel;

0,1-10 % verschiedener Additiva wie Spreitmitteln, Antioxydantien, Farbstoffen, Weichmachern etc.

Zur Herstellung der Formulierungen wird das Gel-, Lack- oder Film-bildende Polymere in einem organischen Lösungs-

Le A 20 522

mittel oder in einem organischen Lösungsmittel/Wasser-Gemisch- oder in Wasser gelöst bzw. angequollen. Gegebenenfalls wird ein Spreitmittel- oder Weichmacher zugesetzt.

Wird eine solche Lösung oder ein solches Gel auf ein nasses oder trockenes Tier gegossen oder gesprüht, so trocknet das wirkstoffhaltige Polymer zu einer Lack- oder Filmschicht ein. Es ist außerdem möglich, mit an Ort und Stelle zur Verfügung stehenden Wirkstoffvormischungen (wettable powder) und dem Polymer eine Art Kleister herzustellen und die Tiere so zu behandeln.

Als Wirkstoffe kommen folgende Insektizide zur Anwendung: aus der Gruppe der Phosphorsäureester z.B. O,O-Diethyl-O-(4-methyl-cumarin-7-yl)-thionophosphorsäureester, O,O-Dimethyl-O-(4-methylmercapto-3-methyl-phenyl)-thiophosphorsäureester, O,O-Dimethyl-(1-hydroxy-2,2,2-trichlorethyl)-phosphorsäureester, O-(1,2-Dibrom-2,2-dichlor-ethyl)-O,O-dimethyl-phosphorsäureester, (O,O-Dimethyl-O-(4-brom-2,5-dichlorphenyl)-thionophosphorsäureester, O,O-Trimethyl-O-(4-dimethylaminosulfonyl-phenyl)-thionophosphorsäureester, und O,O-Diethyl-O-(2-isopropyl-4-methyl-pyrimid-6-yl)-thionophosphorsäureester; aus der Gruppe der Carbamate z.B. Propoxur, Croneton, Carbonyl; außerdem chlorierte insektizide Verbindungen wie chloriertes Camphen mit einem Chlorgehalt von 67-69 % (Toxaphen), Formamidin und dessen Derivate z.B. N-Methyl-N'-2,4-xylol-N-(N-2,4-xylylforminidoyl).

Le A 20 522

Insbesondere eignen sich auch synthetische Pyrethroide, insektizid und akarizid Thioharnstoffe und Amidine (nur als solche mit einer "detaching" - Wirkung).

Die insektiziden Wirkstoffe können in ihrer Wirksamkeit durch Synergisten verstärkt werden. Solche Synergisten sind z.B. Piperonylbutoxid oder Sesamex.

Auch Repellents können in diese Formulierungen eingearbeitet werden, z.B. Diethyltoluamid, Dibutylphthalat, 2-(Octylthio)-ethanol.

Außerdem können "Lockstoffe" der Formulierung zusammen mit Insektiziden beigefügt werden. Die symbovinen Fliegen werden so vermehrt zu einem behandelten Tier gelockt und werden dort getötet, so daß nur ein Teil der Herde behandelt werden muß. Solche Lockstoffe sind z.B.: Vanillin, (Z)-9-Tricosen, (Z)-8-Dodecenylacetat, Octylbutyrat, Eugenol.

Als Gel- und Filmbildner kommen solche makromolekularen Verbindungen in Frage, die sich in Wasser in organischen Lösungsmitteln und in Mischungen organischer Lösungsmittel mit Wasser lösen bzw. anquellen lassen und nach dem Trocknen eine Art Film bilden.

Folgt man einer Einteilung der makromolekularen Hilfsstoffe, wie z.B. von Keipert et al. in Die Pharmazie 28, 145-183 (1973) beschrieben, so kommen vor allem

Le A 20 522

ionische Makromoleküle in deren Salzform zur Anwendung. Dies sind u.a. Natriumcarboxymethylcellulose, Polyacrylsäure, Polymethacrylsäure und deren Salze, Natriumamylopektinsemiglykolat, arabisches Gummi, Xanthan-Gummi und Guar-Gummi.

Amphotere Makromoleküle wie Protein-Derivate, z.B. Gelatine, sind ebenso geeignet wie nichtionische Polymere, z.B. Methylcellulose, Hydroxypropylcellulose und lösliche Stärken, die obige Anforderungen erfüllen.

Als Lösungsmittel sind Wasser und auch alle mit Wasser mischbaren Lösungsmittel geeignet, die das Makromolekül lösen oder anquellen. In Betracht kommen z.B. Alkanole wie Ethanol und Isopropylalkohol, Propylenglykol, Methylcellosolve, Cellosolve, Pyridine, Morpholine, Dioxan, Dimethylsulfoxid, Dimethylformamid, Tetrahydrofuran, Cyclohexanon, t-Butanol-Wasser (9:1), Aceton-Wasser (9:1), Glycerin-Wasser (3:7) oder Toluol-Ethanol (3:2) etc..

Es können bei der Herstellung der erfindungsgemäßen Formulierungen ein oder mehrere Lösungsmittel eingesetzt werden. Als weitere Hilfsmittel sind z.B. spreitende Öle geeignet. Insbesondere sind die folgenden Verbindungsklassen bzw. Verbindungen geeignet.

Le A 20 522

Silikonöle verschiedener Viskosität.

Fettsäureester, wie Ethylstearat, Di-n-butyl-adipat, Laurinsäurehetylester, Dipropylen-glykolpelargonat, Ester einer verzweigten Fettsäure mittlerer Kettenlänge mit gesättigten Fettalkoholen $(C_{16}-C_{18})$, Isopropylmyristat, Isopropylpalmitat, Capryl/Caprinsäureester von gesättigten Fettalkoholen der Kettenlänge $C_{12}-C_{18}$, Isopropylstearat, Ölsäureoleylester, Ölsäuredecylester Ethyloleat, Milchsäureethylester, wachsartige Fettsäureester wie künstliches Entenbürzeldrüsenfett, Dibutylphthalat, Adipinsäurediisopropylester sowie letzterem verwandte Estergemische u.a..

Triglyceride, wie Capryl/Caprinsäuretriglycerid, Triglyceridgemische mit Pflanzenfettsäuren der Kettenlänge $C_8-C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8-C_{10}$-Fettsäuren u.a.

Fettalkohole, wie Isotridecylalkohol, 2-Octyldodecanol, Cetylstearyl-Alkohol, Oleylalkohol.

Fettsäuren, wie z.B. Ölsäure.

Besonders gut geeignete spreitende Öle sind die folgenden: Isopropylmyristat, Isopropylpalmitat, Capryl/Caprinsäureester von gesättigten Fettalkoholen der

Le A 20 522

Kettenlänge $C_{12}$-$C_{18}$, wachsartige Fettsäureester wie künstliche Entenbürzeldrüsenfett Silikonöle und Isorpropylmyristat-Isopropylpalmitat-Isopropylstearat-Gemisch.

Als weitere Hilfsmittel sind geeignet:

a.  Substanzen, die z.B. eine Suspension stabilisieren können, z.B. kolloidale Kieselsäure, Montmorillonite u.a.

b.  Tenside (beinhaltet Emulgatoren und Netzmittel), z.B.

1.  anionaktive, wie Na-Laurylsulfat, Fettalkohol-ethersulfate, Mono/Dialkylpolylgykolether-orthophosphorsäureester-Monoethanolaminsalz;

2.  kationaktive, wie Cetyltrimethylammoniumchlorid;

3.  ampholytische, wie Di-Na-N-lauryl-ß-iminodipropionat oder Lecithin;

4.  nicht ionogene, z.B. polyoxyethyliertes Rizinusöl, polyoxyethyliertes Sorbitan-Monooleat, Sorbitan-Monostearat, Cetylalkohol, Glycerin-monostearat, Polyoxyethylenstearat, Alkylphenol-polyglykolether.

c.  Stabilisatoren zur Verhinderung des bei einigen Wirkstoffen eintretenden chemischen Abbaues wie Antioxydantien, z.B. Tocopherole, Butylhydroxy-anisol;

Le A 20 522

d.   Weichmacher, z.B. Propylenglykol, Glycerin, Di-
     und Tripropylenglykol, Triethanolamin, Wachse.

Le A 20 522

Beispiel

Demonstration der Haftwirkung am Tier

Zwei Formulierungen wurden mit gelbem Eisenoxid zur Farb-Markierung nach folgender Zusammensetzung herge- stellt:

| | 1 | 2 |
|---|---|---|
| Hydroxypropylcellulose (M.G. 60.000) | 10,0 g | 10,0 g |
| Eisenoxidgelb | 2,0 g | 2,0 g |
| Isopropanol ad | 100 ml | - |
| Wasser ad | - | 100 ml |

Es wurden zwei Rinder völlig durchnäßt. Obige Suspensionen wurden einmal aufgegossen und einmal mit einer Gartenspritze aufgesprüht. Nach dem Trocknen der Tiere bildet sich eine fest haftende Masse. Die Tiere wurden nun 1 mal täglich längere Zeit mit dem Schlauch erneut "beregnet". Nach 8 Tagen war immer nocht deut- lich die Haftmasse zu sehen.

Le A 20 522

## Patentansprüche

1. Mittel zur Bekämpfung tierischer Ektoparasiten mit ausgeprägter Residual-Wirkung, das sowohl auf nasse wie trockene Tiere aufgetragen werden kann, dadurch gekennzeichnet, daß es 0,1-20 Gew.% eines ektoparasitiziden, insbesondere insektiziden und/oder akariziden Wirkstoffs, 1-40 Gew.% eines in Wasser, in organischen Lösungsmitteln oder in Mischungen aus organischen Lösungsmitteln und Wasser löslichen oder anquellbaren Gel-, Lack- oder Film-bildenden Polymers, 40-98 Gew.% eines organischen wassermischbaren Lösungsmittels, ein Gemisch organischer Lösungsmittel/Wasser oder Wasser und 0,1-10 Gew.% verschiedener Additiva, wie Spreitmittel, Lösungsvermittler, Suspendierhilfsmittel und/oder Antioxydantien und/oder Farbstoffe enthält.

2. Mittel zur Bekämpfung tierischer Ektoparasiten nach Anspruch 1, dadurch gekennzeichnet, daß es einen Repellent-Wirkstoff enthält.

3. Mittel gemäß Anspruch 1 dadurch gekennzeichnet, daß es sich bei dem ektoparasitiziden Wirkstoff um ein Insektizid oder eine Insektizid-Mischung handelt.

4. Mittel gemäß Anspruch 1, dadurch gekennzeichnet, daß es ein Pyrethroid enthält.

5. Mittel gemäß Anspruch 1, dadurch gekennzeichnet, daß es einen insektiziden Phosphorsäureester enthält.

6. Mittel gemäß Anspruch 2, dadurch gekennzeichnet, daß Diethyltoluamid, Dibutylphthalat und/oder 2-(Octylthio)-ethanol enthält.

Le A 20 522

7. Mittel gemäß Anspruch 1, dadurch gekennzeichnet, daß es als Spreitmittel Isopropylmyristat, Isopropylpalmitat, Capryl/Caprinsäureester von gesättigten Fettalkoholen der Kettenlänge $C_{12} - C_{18}$, wachsartige Fettsäureester wie künstliches Entenbürzeldrüsenfett, Silikonöle oder ein Isopropylmyristat-Isopropylpalmitat-Isopropylstearat-Gemisch enthält.

8. Verfahren zur Herstellung von Mitteln zur Bekämpfung tierischer Ektoparasiten, dadurch gekennzeichnet, daß man ektoparasitizide Wirkstoffe bzw. Repellent-Wirkstoffe, ein Gel-, Lack- oder Film-bildendes Polymer in Wasser oder einem Lösungsmittel oder in einem Wasser/Lösungsmittelgemisch, gegebenenfalls in Gegenwart weiterer Hilfsstoffe miteinander vermischt.

9. Verfahren zur Herstellung von Mitteln zur Bekämpfung tierischer Ektoparasiten nach Anspruch 4, dadurch gekennzeichnet, daß man 0,1 bis 20 Gewichtsprozent ektoparasitizide Wirkstoffe bzw. Repellent-Wirkstoffe, 1 bis 50 Gewichtsprozent eines Gel-, Lack- oder Film-bildenden Polymers, 0 bis 10 Gewichtsprozent eines oder mehrerer weiterer Hilfsstoffe und 40 bis 98 Gewichtsprozent Wasser und/oder Wasser/Lösungsmittelgemisch oder organisches Lösungsmittel miteinander vermischt.

10. Verfahren zur Bekämpfung tierischer Ektoparasiten, dadurch gekennzeichnet, daß man ein Mittel gemäß Anspruch 1 auf nasse oder auf trockene Tiere appliziert, die unter Ektoparasitenbefall leiden.

Le A 20 522

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| E,X | EP - A - 0 041 654 (BAYER)<br><br>* Seite 6, Zeilen 6-8; Ansprüche *<br><br>-- | 1-6, 8-10 |
| X | US - A - 4 199 564 (J.SILVER et al.)<br><br>* Spalte 4, Zeilen 54-65; Ansprüche 1,7,8 *<br><br>-- | 1,8, 10 |
| X | FR - A - 2 216 921 (PEPRO)<br><br>* Seite 2, Zeilen 8-28; Seite 3, Zeilen 7-40; Ansprüche 1;5,7 *<br><br>-- | 1,3-5, 8-10 |
| X | GB - A - 802 111 (I.I.LUBOWE)<br><br>* Seite 1, Zeilen 54-74; Ansprüche 1,4,26 *<br><br>-- | 1,3-5, 7-10 |
| Y | GB - A - 1 108 837 (ASTRA)<br><br>* Seite 2, Zeilen 5-34; Anspruch *<br><br>-- | 1-10 |
| D,Y | DE - A - 2 658 725 (BAYER)<br><br>* Ansprüche; Seite 8 *<br><br>-- | 1-10 |
| A | GB - A - 811 135 (BASF)<br><br>* Seite 3, Zeilen 5-9 *<br><br>-- ./. | 1-10 |

**KLASSIFIKATION DER ANMELDUNG (Int. Cl.3)**

A 01 N 25/24
A 61 K 7/40

**RECHERCHIERTE SACHGEBIETE (Int. Cl.3)**

A 01 N 25/24
A 61 K 7/40
A 61 L 15/06

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| DEN HAAG | 16-02-1982 | PELTRE |

EPA form 1503.1  06.78

Europäisches
Patentamt

EUROPÄISCHER RECHERCHENBERICHT

0051786

Nummer der Anmeldung

EP 81 10 8892

-2-

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| Y | CH - A - 423 353 (COOPER,McDOUGALL & ROBERTSON)<br><br>* Seite 2, Zeilen 78-101; Ansprüche *<br><br>----------- | 1-10 | |

RECHERCHIERTE SACHGEBIETE (Int. Cl.³)